# EUROPÄISCHE PATENTSCHRIFT

(11) **EP 0 416 353 B1**
(45) Veröffentlichungstag und Bekanntmachung des Hinweises auf die Patenterteilung: **09.08.1995**
(21) Anmeldenummer: 90115846.9
(22) Anmeldetag: 18.08.1990
(51) Int. Cl.: A61M 5/20

(54) **Injektionsspritze**
Syringe
Seringue à injection

(30) Priorität: 07.09.1989 DE 3929777
(43) Veröffentlichungstag der Anmeldung: 13.03.1991
(73) Patentinhaber: Lucas, Dieter, Dr., D-78337 Öhningen (DE)
(72) Erfinder: Lucas, Dieter, Dr., D-78337 Öhningen (DE)
(74) Vertreter: Hiebsch, Gerhard F., Dipl.-Ing.

(56) Entgegenhaltungen:
- CH-A- 554 174
- DE-A- 3 417 757
- FR-A- 1 538 565
- GB-A- 1 528 735
- US-A- 3 556 100
- US-A- 4 648 872

## Beschreibung

Die Erfindung betrifft eine Injektionsspritze nach dem Oberbegriff des Patentanspruches 1.

Es gibt Wegwerfspritzen, bei denen die Nadel zur Funktionsfähigkeit auf die Spritze aufgeschraubt werden muß. Die Injektionsflüssigkeit wird aus Ampullen entnommen und in die Spritze aufgezogen. Die Manipulation mit derartigen Spritzen ist hygienisch nicht einwandfrei und darüber hinaus zeitraubend.

Demgegenüber stellt die Injektionsspritze nach der dem Anspruch 1 zugrundeliegenden DE-A1-34 17 757 des Erfinders eine Verbesserung dar. Deren Flüssigkeitsraum weist eine zum Nadelraum hin abragende Ausformung auf, in welche in Betriebsstellung ein stiftartiger Teil der Injektionsnadel einragt. Auf dem Spritzkolben kann eine Druckplatte oder eine entsprechende Kolbenscheibe mit axialem Kolbenrohr ruhen, in welchem eine von außen zugängliche Kolbenstange des Spritzkolbens verschieblich lagert und welches von einem Arretierungsorgan in Bereitschaftsstellung gehalten wird.

Bei einer Injektionsspritze nach FR-A-1 538 565 sind Spritzgutkammer und Nadelraum in komplizierter Weise mit der Injektionsnadel koaxial ineinander angeordnet. Der behandelnde Arzt kann die Bewegung der federbelasteten Spritze nicht steuern.

Die US-PS 3 556 100 zeigt eine Spritze, bei welcher sich Injektionsnadel und Injektionsflüssigkeit in einem gemeinsamen Raum befinden. Unter dem Druck einer Schraubenfeder wird die Nadel aus dem Raum gestoßen und gleichzeitig die Flüssigkeit durch eine Nadelkanüle gepreßt. Diese Anordnung hat zum einen den Nachteil, daß Nadel und Flüssigkeit in ständigem Kontakt miteinander stehen, wobei Korrosionserscheinungen an der Nadel wegen der oftmals aggressiven Flüssigkeiten oder Verunreinigungen der Flüssigkeit nicht ausgeschlossen sind. Zum anderen wird in jedem Fall mit der Nadel auch die Flüssigkeit freigesetzt, so daß es nicht möglich ist, zuerst die Nadel beispielsweise in das Muskelgewebe eines Patienten einzubringen und dann die Zugabe der Flüssigkeit vorzunehmen.

Die zunehmende Sorge um Folgen einer Verletzung Dritter durch bereits benützte Spritzen führt zur Aufgabe, deren Nadeln nach Gebrauch weitestgehend unschädlich werden zu lassen sowie die Überführung der Injektionsnadel aus der Bereitschaftsstellung in die Betriebsstellung u.zurück, also die Handhabung, in einfacher Weise erfolgen soll. Zur Lösung dieser Aufgabe fuhrt die Lehre des unabhängigen Anspruches; die Unteransprüche geben günstige Weiterbildungen an.

Erfindungsgemäß ist die Injektionsnadel in Betriebsstellung von einem Riegel gehalten sowie aus der Betriebsstellung unter Freigabe durch den Riegel mittels eines Kraftspeichers in den Aufnahmeraum rückführbar, wobei zwischen dem federbelasteten Kolben und dem Boden des Aufnahmeraumes ein sich radial erstreckendes Rastorgan axial beweglich angeordnet ist, das in Bereitschaftsstellung gegen den bodenwärts abgestützten Kraftspeicher gehalten ist und beim Überführen der Injektionsnadel in die Betriebsstellung den Kolben arretiert. Dank dieser Maßgabe kann die rückholbare Spritze während der Handhabung fixiert bleiben, so daß eine genaue Einführung erfolgen kann.

Im Rahmen der Erfindung liegt eine Injektionsspritze, deren Nadelraum für die Injektionsnadel in einer im Aufnahmeraum axial bewegbaren Büchse vorgesehen ist. Hier ist das Rastorgan in der in Betriebsstellung vom Riegel beaufschlagten, einen Nadelraum für die Injektionsnadel umfassenden Büchse als Arretierung für den Kolben angebracht.
Nach einem anderen Merkmal der Erfindung ist die Büchse mit einem in Betätigungsrichtung spannbaren Kraftspeicher verbunden. Dabei ist die Injektionsnadel bzw. ihr Kolben im Nadelraum vorteilhaft durch eine dann gespannte Feder gehalten, Büchse und Injektionsnadel hängen an einer Spanneinrichtung, bei deren Freigabe Büchse und Injektionsnadel in eine Druckrichtung geschossen werden; in Betriebsstellung ist die Büchse nach einem weiteren Merkmal der Erfindung so verriegelt, daß die Injektionsnadel aus dem Aufnahmeraum ragt.

Der bodenwärtige Kraftspeicher stützt sich im Aufnahmeraum gegen eine Außenlippe, welche nahe dem bodenwärtigen Büchsenrand an der Büchse verläuft und in Verschlußlage (Bereitschaftstellung) -- also bei eingezogener bzw. umhüllter Injektionsnadel -- zwischen einer Verschlußplatte des Aufnahmeraums sowie einer innenliegenden Rastrippe des Aufnahmeraumes ruht.

Die Büchse selbst weist einen inneren Radialkragen od. dgl. Innenlippe auf, der/die in Betriebsstellung der Injektionsspritze von dieser untergriffen wird. Dank des Radialkragens wird der Kolben in der Büchse sowohl in Betriebsstellung als auch in Verschlußlage so gehalten, daß er den Büchsenboden bildet. Eine Paarung einander zugekehrter Radialorgane von Büchse und Aufnahmeraum gewährleistet erfindungsgemäß einen festen Sitz der Büchse -- und damit der Injektionsnadel -- in der Verschlußlage; bei allen beschriebenen Ausführungen ist die Injektionsnadel in einfacher Weise aus der Bereitschaftsstellung in die Betriebsstellung überführbar und kann nach Gebrauch ohne weiteres außer Zugriff in ein Gehäuseteil eingezogen werden.

Als günstig hat es sich auch erwiesen, das Rastorgan als ein von der Injektionsnadel durchsetzbaren Ring zu gestalten, der von wenigstens einer lösbaren Riegeleinrichtung in Bereitschafts- und Betriebsstellung gegen eine Feder gehalten wird. Den Ring hintergreifen als Gegenrasten flexible Hakenelemente des Kolbens, die bei dessen Übergang aus der Betriebsstellung in eine Verschlußlage den Ring mitführen, d. h. das Rastorgan bzw. der Ring wird bei Freigabe durch die Riegeleinrichtung mit dem anliegenden Kolben durch den Kraftspeicher zur Verschlußplatte des Nadelraums gedrückt. Die Riegeleinrichtung -- bevorzugt wenigstens ein achsparallel zur Injektionsnadel verlaufender Riegelstab -- kann mit ihrem freien Ende an ein etwa radial bewegbares Kulissengestänge angeschlossen sein, welches sie schlagartig freisetzt, wenn der Spritzvorgang beendet ist und die Injektionsnadel in das Gehäuse zurückkehren soll.

Weitere Vorteile und Einzelheiten der Erfindung ergeben sich aus der nachfolgenden Beschreibung bevorzugter Ausführungsbeispiele sowie anhand der Zeichnung; diese zeigt in:
- Fig. 1:: eine Injektionsspritze mit zwei parallel nebeneinanderliegenden Kammern und einer Injektionsnadel in Bereitschaftsstellung;
- Fig. 2:: die Injektionsspritze nach Fig. 1 in Betriebsstellung;
- Fig. 3:: ein vergrößertes Detail der Fig. 2;
- Fig. 4:: die Injektionsspritze der Fig. 1,2 in entleertem Zustand;
- Fig. 5:: einen Teil einer anderen Injektionsspritze in Bereitschaftsstellung;
- Fig. 6:: die Injektionsspritze nach Fig. 5 in Betriebsstellung;
- Fig. 7:: die Injektionsspritze nach Fig. 5,6 in entleertem Zustand;
- Fig. 8:: Details eines spezifischen Verschlusses an der Injektionspritze.

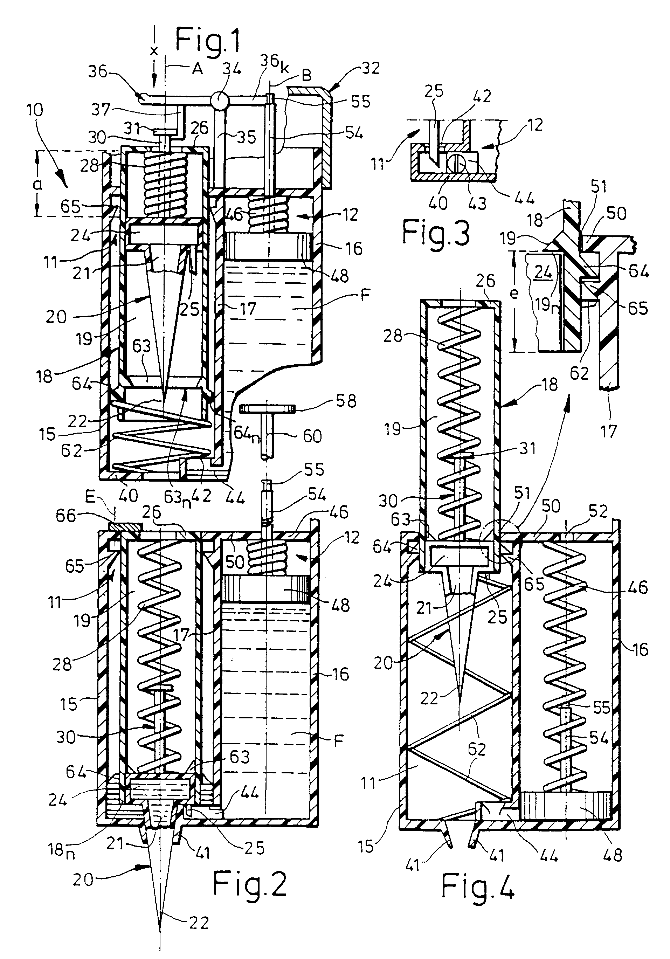

Die in Fig. 1 dargestellte Ausführung einer Injektionsspritze 10 für flüssige Medikamente besteht aus zumindest zwei zylindrischen Aufnahmeräumen 11,12 in aneinandergefügten Gehäusen 15,16 mit parallelen Längsachsen A,B. In dem in Fig. 1 linken Aufnahmeraum 11 ist eine koaxiale zylindrische Büchse 18 axial verschieblich, in der eine Hohlnadel 20 in dargestellter Bereitschaftsstellung so untergebracht ist, daß deren Nadel spitze 22 innerhalb der Büchse 18 liegt. Dabei steht ein die Hohlnadel 20 tragender, im Nadelraum 19 der Büchse 18 geführter Hohlkolben 24 in einem Abstand a zu einer Deckplatte 26 der Büchse 18, gegen die sich eine Schraubenfeder 28 mit ihrem oberen Ende abstützt; deren unteres Ende lastet auf dem Hohlkolben 24. Die Schraubenfeder 28 wird von einem Kolbenstab 30 mit radial abragendem Kolbenkopf 31 durchsetzt, der in Fig. 1 an der Büchse 18 ragt und von einer Arretiereinrichtung gehalten wird.

Nach dem Abnehmen einer beiden Gehäusen 15,16 gemeinsam zugeordneten Schutzkappe 32 wird durch Druck (Pfeil x) auf einen bei 34 an einen Bolzen 35 angelenkten Verschlußhebel 36 der Kolbenkopf 31 und damit der Kolbenstab 30 freigegeben; der Hohlkolben 24 wird von der Schraubenfeder 28 abwärts zum Gehäuseboden 40 hin geführt, wobei durch einen exzentrischen Nadelstutzen 25 eine seitliche Verriegelung 42 od. dgl. im Aufnahmeraum 11 an der Mittelwand 17 zerstört wird; diese verschließt einen mit Ventilkugel 43 versehenen Durchgangsraum 44 zum benachbarten Aufnahmeraum 12, welcher in der Betriebsstellung nach Fig. 2 über den Nadelstutzen 25 mit dem Hohlkolben 24 und dadurch mit dem Nadelhohlraum 21 verbunden wird. In letzteren strömt sofort unter Druck eines von einer Druckfeder 46 belasteten Druckkolbens 48 Flüssigkeit F aus dem als Spritzgutkammer dienenden Aufnahmeraum 12. Die Druckfeder 46 ist in Bereitschaftsstellung der Injektionsspritze 10 zwischen dem Druckkolben 48 und einer beiden Gehäusen 15,16 gemeinsamen Verschlußplatte 50 gespannt. Diese weist Durchbrüche 51 bzw. 52 unterschiedlicher Durchmesser für die Büchse 18 einerseits bzw. einen Führungsstab 54 des Druckkolbens 48 auf. Ein Hakenkopf 55 dieses Führungsstabes 54 wird in Bereitschaftsstellung vom kürzeren Hebelarm 36ₖ jenes Verschlußhebels 36 untergreifend gehalten, der gleichzeitig mittels eines nach unten ragenden Hakenarms 37 des längeren Hebelarms 36ᵢ den Kolbenkopf 31 festlegt. Bei Betätigung des Verschlußhebels 36 in Pfeilrichtung x werden Kolbenstab 30 und Führungsstab 54 -- und damit die Schraubenfedern 28,46, wie oben bereits angedeutet -- freigesetzt und zwar der Führungsstab 54 mit Zeitverzögerung gegenüber dem Absenken des Kolbenstabes 30, so daß der Druck- oder Flüssigkeitskolben 48 etwas später abwärts gelangt. Diese Zeitverzögerung kann beispielsweise durch nicht näher dargestellte Kulissenelemente erzeugt werden. Um ein langsames Verschieben des Druckkolbens 48 zu ermöglichen, kann dessen Führungsstab 54 noch mit einem Handgriff 58 verbunden werden, welcher seinerseits einen Schaft 60 aufweist.

Im übrigen durchstößt die Injektionsnadel 20 beim Übergang in die Betriebsstellung (Fig. 2) den Gehäuseboden 40, wobei sich dessen Reste als Manschette 41 der Injektionsnadel 20 seitlich anschmiegen.

Die Büchse 18 wird in ihrer oberen Bereitschaftsstellung durch die fixierte Schraubenfeder 28 gehalten sowie bei der schlagartigen Freigabe der Schraubenfeder 28 mit der Hohlnadel 20 abwärts getrieben, wobei der Hohlkolben 24 eine ringartige Innenlippe 63 dreieckförmigen Querschnitts aus flexiblem Material durchfährt und deren radiale Unterfläche 63ₙ hintergreift. Deren Abstand e vom unteren Büchsenrand 18ₙ entspricht der Kolbenhöhe.

Eine untere Rückholfeder 62, die zwischen dem Gehäuseboden 40 und einer ringförmigen Außenlippe 64 der Büchse 18 vorgespannt lagert, wird beim Absenken der Büchse 18 weitergehend gespannt. Die Außenlippe 64 kragt mit ihrer radialen Unterfläche 64ₙ etwas unterhalb der anderen Lippenunterfläche 19ₙ von der Büchse 18 ab.

Während des Injektionsvorganges hält ein bei 66 nur angedeuteter, in die Bewegungsbahn der Büchse 18 um eine Achse E geschwenkter Schnappriegel die Büchse 18 in Betriebsstellung. Wird der Schnappriegel 66 gelöst, drückt jene Rückholfeder 62 die Büchse 18 aufwärts in eine in Fig. 4 gezeigte Endlage, in welcher obere Rastrippen 65 des Gehäuses 15 von der Außenlippe 64 der Büchse 18 überfahren sind; letztere ist zwischen den radialen Oberflächen 65ₕ, Rastrippen 65 und der Verschlußplatte 50 festgeklemmt, die Injektionsnadel 20 damit innerhalb des Aufnahmeraumes 11 fixiert. Eine Verletzung durch die Injektionsspritze 10 wird somit ausgeschlossen.

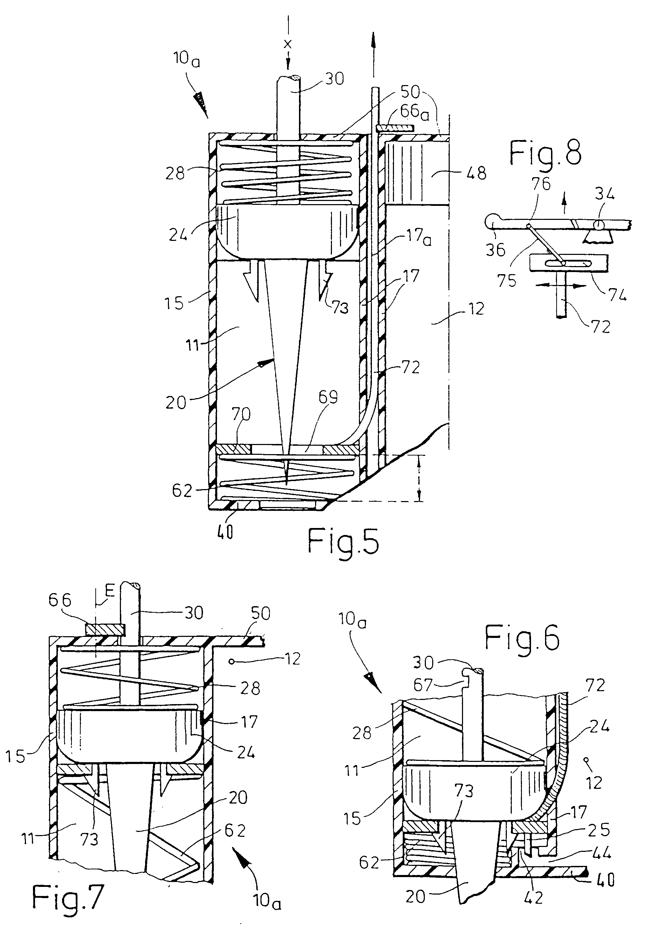

Beim Ausführungsbeispiel der Fig. 5 bis 7 einer Injektionsspritze 10ₐ werden der Hohlkolben 24 und die Injektionsnadel 20 in deren Bereitschaftsstellung durch ein nicht gezeigtes Arretiersystem des Kolbenstabes 30 gehalten. Die obere Schraubenfeder 28 lagert hier zwischen Hohlkolben 24 und Verschlußplatte 50 im Nadelraum 19 des Gehäuses 15; eine Büchse 18 gibt es hier nicht.

Die Rückholfeder 62 ist zwischen dem Gehäuseboden 40 und einem Druckring 70 gespannt, der durch Druckhebel 72 in einem Abstand i zum Gehäuseboden 50 gehalten wird. Der Druckhebel 72 verläuft in einem Kanal 17ₐ der Mittelwand 17 und ist oberhalb der Verschlußplatte 50 betätigbar.

Vom Hohlkolben 24 ragt bodenwärts ein Kranz von begrenzt flexiblen Rasthaken 73 ab, die beim Absenken des Hohlkolbens 24 (Pfeil x) die zentrische Öffnung 69 des Druckrings 70 durchfahren und diesen mit ihren Hakenköpfen hintergreifen; die Hohlnadel 20 ist in Betriebsstellung (Fig. 6). Die Rasthaken 73 gewährleisten zudem die Mitnahme des Druckringes 70 in die Verschlußlage der Injektionsnadel 20.

Nach Gebrauch der Injektionsspritze 10ₐ werden die Druckhebel 72 gelöst, und die Rückholfeder 62 drückt den Hohlkolben 24 aufwärts, bis sein Kolbenstab 30 gemäß Fig. 7 durch Eingriff des Schnapphebels 66 in eine Riegelkerbe 67 arretiert zu werden vermag. In Abhängigkeit von ihrer Federkraft reicht sogar die Rückholfeder 62 zur gesicherten Fixierung der Injektionsnadel 20 im Gehäuse 15 aus.

Der vertikale Druckhebel 72 kann gemäß Fig. 8 mit einem dem quer verlaufenden Kulissenschlitz 74 ausgestattet sein, in dem sich radial zum Druckhebel 72 ein geneigter Lenker 75 bewegen kann, der andernends am Verschlußhebel 36 bei 76 angelenkt ist. Beim Anheben des Verschlußhebels 36 wird der Lenker 75 verschoben und gibt jenen Druckhebel 72 frei.

## Patentansprüche

1. Injektionsspritze mit einer aus einem Aufnahmeraum (11) durch dessen Boden (40) aus einer Bereitsschaftsstellung durch eine Feder (28) in eine Betriebsstellung ausschiebbaren Injektionsnadel (20), mit einem neben dem Aufnahmeraum (11) angeordneten, geschlossenen Behälter (12) mit einer Spritzgutkammer (16), die eine von einem belastbaren Druckkolben (48) unter Druck setzbare Injektionsflüssigkeit (F) aufnimmt, wobei die einen Nadelkanal (25) aufweisende Injektionsnadel (20) an einem federbelasteten Kolben (24) vorgesehen und in Betriebsstellung mit der Spritzgutkammer (16) verbunden ist,
dadurch gekennzeichnet,
daß die Injektionsnadel (20) in Betriebsstellung von einem Riegel (66,66a) gehalten ist sowie aus der Betriebsstellung unter Freigabe durch den Riegel (66,66a) mittels eines Kraftspeichers (62) in den Aufnahmeraum (11) rückführbar ist, wobei zwischen dem federbelasteten Kolben (24) und dem Boden (40) des Aufnahmeraumes (11) ein sich radial erstreckendes Rastorgan (63,70) axial beweglich angeordnet ist, das in Bereitschaftsstellung gegen den bodenwärts abgestützten Kraftspeicher (62) gehalten ist und beim Überführen der Injektionsnadel (20) in die Betriebsstellung den Kolben (24) arretiert.

2. Injektionsspritze nach Anspruch 1, dadurch gekennzeichnet, daß das Rastorgan (63) in einer in Betriebsstellung vom Riegel (66) beaufschlagten, einen Nadelraum (19) für die Injektionsnadel (20) umfassenden Büchse (18) als Arretierung für den Kolben (24) angebracht ist, wobei die Büchse (18) im Aufnahmeraum (11) axial verschieblich lagert.

3. Injektionsspritze nach Anspruch 1, dadurch gekennzeichnet, daß das Rastorgan ein von der Injektionsnadel (20) durchsetzbarer Ring (70) im Aufnahmeraum (11) ist und von wenigstens einer lösbaren Sperreinrichtung (72) des Riegels (66a) gegen den Kraftspeicher (62) gehalten ist.

4. Injektionsspritze nach Anspruch 3, dadurch gekennzeichnet, daß flexible Hakenelemente (73) des Kolbens (24) den Ring (70) als Gegenrasten in Druckrichtung (x) hintergreifen.

5. Injektionsspritze nach Anspruch 2, dadurch gekennzeichnet, daß die Feder (28) zwischen dem Kolben (24) und einer Deckplatte (26) der Büchse (18) angeordnet ist.

6. Injektionsspritze nach Anspruch 1 oder 2 oder 5, dadurch gekennzeichnet, daß die Büchse (18) durch den bodenwärtigen Kraftspeicher (62) im Aufnahmeraum (11) in ihrer Bereitschaftsstellung gehalten ist.

7. Injektionsspritze nach Anspruch 2 oder 6, dadurch gekennzeichnet, daß der Kraftspeicher (62) gegen eine Außenlippe (64) der Büchse (18) abgestützt ist, welche gegebenenfalls nahe dem bodenwärtigen Büchsenrand (18n) der Büchse (18) verläuft und in Bereitschaftsstellung der Injektionsnadel (20) zwischen einer Verschlußplatte (50) des Aufnahmeraumes (11) sowie einer innenliegenden Rastrippe (65) des Aufnahmeraumes (11) sitzt.

8. Injektionsspritze nach Anspruch 2 oder 7, dadurch gekennzeichnet, daß das Rastorgan (63) und die Außenlippe (64) beidseits der Büchsenwand einander benachbart sind.

## Claims

1. Injection syringe with an injection needle (20) which can be pushed out from a receiving chamber (11) through the base (40) of the latter from a stand-by position into an operational position by means of a spring (28), with a closed container (12) arranged beside the receiving chamber (11) with an injection material chamber (16), which takes up an injection liquid (F) which can be pressurised by a load bearing plunger (48,) while the injection needle (20) comprising a needle channel (25) is provided on a spring-loaded piston (24)and in its operational position is connected to the injection material chamber (16), characterised in that the injection needle (20) in operational position is held by a bar (66, 66a) and is retractable from the operational position into the receiving chamber (11), on release by the bar (66, 66a), by means of a force storage means (62), while between the spring loaded piston (24) and the base (40) of the receiving chamber (11) a radially extending stopping element (63, 70) is arranged in an axially movable manner which in stand-by position is held against the force storage means (62) braced towards the base and arrests the piston (24) when transferring the injection needle (20) into the operating position.

2. Injection syringe according to claim 1,
characterised in that the stopping element (63) is mounted as arrest for the piston (24) in a barrel (18) surrounding a needle chamber (19) for the injection needle (20) and acted on by the bar (66) in operating position, the barrel (18) resting in an axially displaceable manner in the receiving chamber (11).

3. Injection syringe according to claim 1,
characterised in that the stopping element is a ring (70) in the receiving chamber (11) penetrable by the injection needle (20) and is held by at least one releasable locking device (72) of the bar (66a) against the force storage means (62).

4. Injection syringe according to claim 3,
characterised in that flexible hook elements (73) of the piston (24) catch behind the ring (70) as counterstops in the direction of pressure (x).

5. Injection syringe according to claim 2,
characterised in that the spring (28) is arranged between the piston (24) and a cover plate (26) of the barrel (18).

6. Injection syringe according to claim 1 or 2 or 5,
characterised in that the barrel (18) is held in its stand-by position by the force storage means (62) towards the base in the receiving chamber (11).

7. Injection syringe according to claim 2 or 6,
characterised in that the force storage means (62) is braced against an outer lip (64) of the barrel (18) which if need be extends close to the barrel edge (18n) facing the base of the barrel (18) and in the stand-by position of the injection needle (20) sits between a closure plate (50) of the receiving chamber (11) and an internally located stop rib (65) of the receiving chamber (11).

8. Injection syringe according to claim 2 or 7,
characterised in that the stopping element (63) and the outer lip (64) are adjacent to each other on both sides of the barrel wall.

## Revendications

1. Seringue à injection comprenant une aiguille d'injection (20), laquelle peut être coulissée d'une position d'attente à une position active sous l'effet d'un ressort (28) qui pousse l'aiguille au travers du fond (40) d'un logement (11) et comprenant à côté du logement (11) un récipient (12) contenant un réservoir de liquide d'injection (F) pouvant être mis sous pression par un piston plongeur (48) chargeable, l'aiguille d'injection (20) présentant un canal (25) étant solidaire d'un piston (24) poussé par un ressort et étant reliée en position active avec la chambre de liquide d'injection (16),
caractérisée en ce que l'aiguille d'injection (20) est maintenue par un verrou (66, 66a) en position active, de même qu'elle peut, par le déverrouillage du verrou (66, 66a) quitter la position active et reprendre sa position dans le logement (11) au moyen d'un accumulateur d'énergie (62), un organe d'arrêt (63, 70) s'étendant dans la direction radiale étant disposé de manière à se déplacer axialement entre le piston chargé par un ressort (24) et le fond (40) du logement, cet organe d'arrêt étant maintenu en position d'attente contre l'accumulateur d'énergie (62) s'appuyant sur le fond et arrêtant le piston (24) dans la position active, lors du coulissement de l'aiguille d'injection (20).

2. Seringue d'injection selon la revendication 1, caractérisée en ce que l'organe d'arrêt (63) est placé dans une douille (18) entourant l'espace (19) prévu pour l'aiguille d'injection (20) et est retenu par un verrou (66) en position active, cet organe d'arrêt étant prévu pour arrêter le piston (24), ladite douille (18) étant disposée de manière à pouvoir coulisser axialement dans le logement (11).

3. Seringue à injection selon la revendication 1, caractérisée en ce que l'organe d'arrêt est un anneau (70) prévu dans le logement (11) et pouvant être traversé par l'aiguille d'injection (20), cet organe d'arrêt étant également maintenu contre l'accumulateur d'énergie (62) par au moins un dispositif de blocage (72) amovible constituant le verrou (66a).

4. Seringue à injection selon la revendication 3, caractérisée en ce que des crochets (73) flexibles du piston (24) saisissent l'anneau (70) comme des arrêts opposés au sens de la pression (X).

5. Seringue à injection selon la revendication 2, caractérisée en ce que le ressort (28) est disposé entre le piston (24) et une plaque de fond (26) de la douille (18).

6. Seringue à injection selon l'une ou l'autre des revendications 1, 2 et 5, caractérisée en ce que la douille (18) est maintenue dans sa position d'attente par l'accumulateur d'énergie (62) placé dans le fond du logement (11).

7. Seringue à injection selon l'une ou l'autre des revendications 2 et 6, caractérisée en ce que l'accumulateur d'énergie (62) est appuyé contre un bord extérieur (64) de la douille (18), ce bord passant le cas échéant à proximité du bord (18n) de la douille (18) dirigé vers le fond et étant placé, en position d'attente de l'aiguille d'injection, (20) entre une plaque de fermeture (50) du logement (11) et une nervure d'arrêt (68) intérieure du logement (11).

8. Seringue d'injection selon l'une ou l'autre des revendications 2 et 7, caractérisée en ce que l'organe d'arrêt (63) et le bord externe (64) sont contigus de part et d'autre de la paroi de la douille.
